# DEMANDE DE BREVET EUROPEEN

(11) **EP 4 049 615 A1**
(43) Date de publication de la demande: **31.08.2022**
(21) Numéro de dépôt: 21305226.9
(22) Date de dépôt: 25.02.2021
(51) Int. Cl.: A61C 8/00, A61C 13/00, A61L 27/10, A61L 27/30, A61L 27/50

(54) **DISPOSITIF MEDICAL IMPLANTABLE COMPRENANT UNE COUCHE DE ZIRCONE, ET SON PROCEDE DE PREPARATION**

(71) Demandeur: Centre National de la Recherche Scientifique, 75016 Paris (FR); Université de Lorraine, 54000 Nancy (FR); Centre Hospitalier Régional et Universitaire de Nancy, 54000 Nancy (FR)
(72) Inventeur: BRAVETTI, Pierre, 54000 Nancy (FR); KOUITAT-NJIWA, Richard, 54410 Laneuville- Devant-Nancy (FR); PIERSON, Gaël, 54520 Laxou (FR); DIDELOT, Aurélien, 54500 Vandoeuvre-Lès-Nancy (FR)
(74) Mandataire: Gevers & Orès

(57) **Abrégé**

La présente invention concerne un dispositif médical implantable comprenant une couche constituée ou comprenant de la zircone, et son procédé de préparation.

## Description

La présente invention concerne un dispositif médical implantable comprenant une couche constituée ou comprenant de la zircone, et son procédé de préparation.

Les implants dentaires sont des racines artificielles qui sont encastrées dans l'os, et qui sont destinés à créer un ancrage capable de recevoir une prothèse dentaire amovible ou fixée.

Les prothèses sur implants sont plus confortables et discrètes que les prothèses amovibles. Elles préservent par ailleurs l'os de la mâchoire et gardent saine la denture existante. La pose d'un bridge, au contraire, nécessite de tailler les dents adjacentes pour lui servir de support, donc une partie de l'émail. Un autre inconvénient du bridge est que l'os autour de la dent manquante va se résorber progressivement. Enfin, par rapport à une prothèse amovible, on note quant aux implants un meilleur confort, une meilleure fonctionnalité, une stabilité et une mastication normale, ainsi qu'une sensation d'appartenance.

L'histoire des implants dentaires commence réellement dans les années 1950, quand le Professeur suédois Per Ingvar Brânemark, considéré comme le père de l'implantologie moderne, découvre l'exceptionnelle affinité du titane pour l'os vivant. Le titane devient alors le premier matériau connu qui soit totalement biocompatible. Le Professeur Brânemark décide ainsi d'utiliser le titane pour traiter les patients édentés, développant à l'époque une vis en titane qui se fixe dans la mâchoire pour servir de support à une prothèse dentaire.

Au contact du titane, l'os va cicatriser et se souder : c'est le phénomène d'ostéointégration qui prend 2 à 6 mois en fonction des cas cliniques. L'implant dentaire devient fonctionnel. Il est utilisé soit comme support d'une prothèse fixe (bridge ou couronne) soit pour stabiliser une prothèse amovible.

Avant que l'utilisation du titane ne se développe, les implants dentaires ne pouvaient pas s'intégrer à l'os de la mâchoire, comme cela est permis aujourd'hui.

Cependant, comme tous les métaux, le titane n'est pas inaltérable en présence d'un milieu biologique tel que la salive, et les produits issus de sa corrosion électrochimique peuvent se révéler toxiques, et donner lieu à des réactions allergiques immédiates ou retardées qui pourraient expliquer les échecs successifs d'implants dentaires qui surviennent chez certains patients. En effet, l'hypersensibilité potentielle aux implants en titane est actuellement un fait reconnu. On observe qu'une partie importante de la population est sensible à la présence de métal en bouche. Cette sensibilité se traduit par divers symptômes, tels que maux de tête, irritation cutanée, altération du goût, problème articulaire ou encore fatigue chronique. Nombre de problèmes de santé liés aux implants ont été attribués à des événements d'oxydation et/ou de corrosion qui surviennent en raison de l'environnement biologique, de la fatigue des matériaux, des contraintes mécaniques, de l'électrogalvanisme, de l'exposition au milieu buccal agressif, de concentrations localement élevées en fluorures, comme c'est le cas avec l'application de gels fluorés, de l'usure des matériaux, ou d'une combinaison de tous ces facteurs.

Plus récemment, sont apparus des implants en zircone. Le problème électrochimique qui est dû au titane n'existe pas avec les implants en céramique d'oxyde de zirconium. La zircone est une biocéramique inerte qui présente d'excellentes propriétés biomécaniques, transmet moins la chaleur que le titane, n'induit aucune réaction galvanique, et contrairement au titane, n'est pas sensible à la corrosion dans le milieu buccal. En l'absence d'électron libre, les céramiques d'oxyde de zirconium sont des isolants électriques et donc totalement exemptes de métal. La zircone n'étant pas un conducteur thermique signifie que les implants peuvent être meulés en bouche sans risque de provoquer une nécrose de l'os. Sa teinte blanche favorise des restaurations esthétiques. La céramique en oxyde de zirconium est ainsi extrêmement biocompatible et n'a semble-t-il aucun impact sur le système immunitaire. De plus, avec la zircone, la plaque bactérienne ne se dépose pas et ceci favorise l'hygiène et la pérennité de la restauration implanto-portée.

Toutefois, le titane reste supérieur à la zircone d'un point de vue mécanique, c'est un matériau ductile avec une ténacité six fois supérieur. Sa limite de rupture est largement supérieure à la zircone. En outre, le prix des implants en zircone reste actuellement plus élevé que celui des implants classiques en titane.

L'invention a donc pour but de proposer des dispositifs médicaux implantables, notamment des implants dentaires, ayant une excellente bio- et immuno-compatibilité tout en conservant une très bonne ostéointégration et des propriétés mécaniques avantageuses.

Un autre but de l'invention est de fournir un procédé de préparation de tels dispositifs médicaux implantables facile à mettre en œuvre, sans modifications importantes par rapport aux procédés d'obtention des implants du commerce.

Encore un autre but de l'invention est de fournir un procédé de préparation de tels dispositifs médicaux implantables, lequel ne modifie pas la rugosité cible des implants du commerce.

Ainsi, selon un premier aspect, l'invention concerne un dispositif médical implantable comprenant sur tout ou partie de sa surface une couche constituée ou comprenant de la zircone, ladite couche ayant une épaisseur entre environ 50 et environ 2000 nm.

De façon surprenante, la couche constituée ou comprenant la zircone des dispositifs médicaux implantables de l'invention, avec son épaisseur spécifique, ne modifie pas ou quasiment pas la rugosité desdits dispositifs. Cette couche a en outre une excellente adhérence à la surface des dispositifs et confère auxdits dispositifs les propriétés avantageuses de la zircone, par exemple son aspect visuel désirable.

Selon un mode de réalisation particulier, la zircone n'est pas en présence de, ou sous la forme d'un alliage comprenant, outre la zircone, de l'aluminium, du titane et/ou du carbone, la zircone n'étant en particulier pas sous la forme d'un alliage zircone-aluminium, par exemple un alliage ATZ, zircone-titane ou zircone-carbone.

Selon un mode de réalisation particulier, ladite couche a une épaisseur comprise entre environ 100 ou 200 et environ 2000 nm, en particulier entre environ 500 ou 600 et environ 800 nm.

Selon un mode de réalisation particulier, l'invention concerne un dispositif tel que défini précédemment, comprenant une couche constituée ou comprenant de la zircone, ladite couche ayant sur une partie de la surface dudit dispositif une épaisseur comprise entre environ 50 et environ 2000 nm, notamment entre environ 50 et environ 1200 nm, et sur une autre partie de la surface dudit dispositif une épaisseur comprise entre environ 50 et environ 2000 nm, notamment entre environ 800 et environ 2000 nm.

Selon un mode de réalisation particulier, la zircone est de la zircone yttriée.

Selon un mode de réalisation particulier, la zircone est partiellement ou totalement dans sa phase quadratique, cubique ou monoclinique, notamment monoclinique.

Selon un mode de réalisation particulier, la couche comprend, outre la zircone, au moins un élément ou composé comprenant un élément choisi parmi Mg, Ca, Ag, et Zn, en particulier sous forme métallique ou d'oxyde.

Le au moins un élément ou composé comprenant un élément choisi parmi Mg, Ca, Ag, et Zn peut être au sein de la couche comprenant la zircone, et/ou à la surface de la couche comprenant la zircone. Dans ce dernier cas, la couche selon l'invention se compose d'une première sous-couche comprenant la zircone et d'une deuxième sous-couche dudit au moins un élément ou composé comprenant un élément choisi parmi Mg, Ca, Ag, et Zn, en particulier sur tout ou partie de la sous-couche comprenant la zircone.

Selon un mode de réalisation particulier, l'élément choisi parmi Mg, Ca, Ag, et Zn, se trouve au moins partiellement à la surface de la couche, notamment sur 1 à 100%, par exemple sur 10 à 30%, de ladite surface.

Selon un mode de réalisation particulier, l'invention concerne un dispositif tel que défini précédemment, lequel, notamment en sa surface, à l'exclusion de ladite couche, est dépourvu de zircone ou d'un composé le comprenant.

Selon un mode de réalisation particulier, l'invention concerne un dispositif tel que défini précédemment, lequel, notamment sa surface, est constitué ou comprend du titane ou de l'aluminium.

Selon un mode de réalisation particulier, le titane est du titane grade 4, ou sous la forme d'un alliage, en particulier le titane grade 5 ou un alliage titane-niobium.

Selon un mode de réalisation particulier, le titane ou l'alliage le contenant a été usiné, notamment étiré à froid, moulé, fritté, imprimé par fabrication additive.

Selon un mode de réalisation particulier, ladite couche a une rugosité Ra comprise de 0 à 4,0 µm, par exemple de 0,01 ou 0,02 à 4,0 µm, notamment de 0,6 à 2,0 µm, en particulier de 1,0 à 1,5 µm.

Le dispositif tel que mentionné ci-dessus peut être tout dispositif médical implantable bien connu de l'homme du métier.

Selon un mode de réalisation particulier, ledit dispositif est un implant, en particulier un implant dentaire, ou une prothèse, en particulier une prothèse de hanche, plus particulièrement une cupule, une prothèse d'épaule, ou une prothèse de genou.

Selon un mode de réalisation particulier, ledit dispositif est un implant dentaire comprenant une zone de contact osseux dont tout ou partie de la surface est recouvert de ladite couche avec une première épaisseur comprise entre environ 50 et environ 2000 nm, notamment entre environ 50 et environ 1200 nm, et une zone de contact gingival dont tout ou partie de la surface est recouvert de ladite couche avec une deuxième épaisseur comprise entre environ 50 et environ 2000 nm, notamment entre environ 800 et environ 2000 nm.

Selon un autre aspect, l'invention concerne également un procédé de préparation d'un dispositif médical implantable comprenant sur tout ou partie de sa surface une couche constituée ou comprenant de la zircone, ladite couche ayant une épaisseur comprise entre environ 50 et environ 2000 nm, ledit procédé comprenant une étape de dépôt, sur tout ou partie de la surface d'un dispositif médical implantable, d'une couche constituée ou comprenant de la zircone, ladite couche ayant une épaisseur comprise entre environ 50 et environ 2000 nm, ledit dépôt étant effectué par dépôt physique en phase vapeur (PVD) ou par projection dynamique par gaz froid (cold spray).

Le dépôt peut se faire par toute technique de dépôt physique en phase vapeur ou de projection dynamique par gaz froid, bien connues de l'homme du métier.

Tout mode de réalisation particulier décrit plus haut à propos du dispositif de l'invention s'applique également ici, seul ou en combinaison.

Selon un mode de réalisation particulier, le dépôt physique en phase vapeur est réalisé par pulvérisation cathodique magnétron.

Selon un mode de réalisation particulier, ledit dispositif médical implantable est en rotation, ou non, autour d'au moins un axe de rotation pendant tout ou partie du dépôt.

Selon un mode de réalisation particulier, le dépôt est effectué par dépôt physique en phase vapeur (PVD), en particulier par pulvérisation cathodique magnétron, et se fait en présence d'une cible en zirconium ou en zirconium yttrié.

Selon un mode de réalisation particulier, le dépôt se fait en présence d'une cible en zirconium et d'une cible d'au moins un élément ou d'un composé comprenant au moins un élément choisi parmi Mg, Ca, Ag, et Zn.

Selon un autre mode de réalisation particulier, le dépôt se fait en présence d'une cible en zirconium, et par mise en contact du dispositif avec au moins un élément ou d'un composé comprenant au moins un élément choisi parmi Mg, Ca, Ag, et Zn, par électrophorèse ou par jet de plasma.

Selon un mode de réalisation plus particulier, ledit au moins un élément ou au moins un composé comprenant au moins un élément choisi parmi Mg, Ca, Ag, et Zn est introduit, en particulier sous forme de nanoparticules, dans le jet de plasma formé au cours du dépôt de zircone.

Selon un autre mode de réalisation plus particulier, ledit d'au moins un élément ou d'un composé comprenant au moins un élément choisi parmi Mg, Ca, Ag, et Zn est déposé de façon concomitante à la zircone.

Selon encore un autre mode de réalisation plus particulier, ledit d'au moins un élément ou d'un composé comprenant au moins un élément choisi parmi Mg, Ca, Ag, et Zn est déposé après que la zircone a été déposée.

Selon un mode de réalisation particulier, la variation entre la rugosité Ra du dispositif initial et la rugosité Ra du dispositif obtenu à l'issue du procédé est inférieure ou égale à environ 25%, par exemple inférieure ou égale à environ 20, 15, 10, 9, 8, 7, 6, 5, 4, 3, 2 ou 1%.

Selon un autre mode de réalisation particulier, la variation entre la rugosité Ra du dispositif initial et la rugosité Ra du dispositif obtenu à l'issue du procédé est inférieure ou égale à environ 1%, par exemple inférieure ou égale à environ 0,9 ; 0,8 ; 0,7 ; 0,6 ; 0,5 ; 0,4 ; 0,3 ; 0,2 ; ou 0,1%.

Selon un mode de réalisation particulier, l'étape de dépôt est précédée d'une étape de décapage, en particulier de décapage plasma, du dispositif.

Cette étape peut permettre, si nécessaire, d'augmenter l'adhérence de la couche constituée ou comprenant la zircone. Le dispositif médical implantable peut être obtenu par toute technique bien connue de l'homme du métier.

Par exemple, le dispositif médical implantable, notamment un implant dentaire, peut être obtenu par usinage, notamment étirage à froid, moulage, frittage, impression par fabrication additive, ou toute autre technique ad hoc bien connue de l'homme du métier.

Selon un autre aspect, l'invention concerne un dispositif médical implantable susceptible d'être obtenu selon le procédé tel que défini ci-dessus.

### Définitions

Tel qu'on l'entend ici, les plages de valeur sous forme de « x-y » ou « de x à y » ou « entre x et y » incluent les bornes x et y ainsi que les entiers compris entre ces bornes. A titre d'exemple, « 1-5 », ou « de 1 à 5 » ou « entre 1 et 5 » désignent les entiers 1, 2, 3, 4 et 5. Les modes de réalisations préférés incluent chaque entier pris individuellement dans la plage de valeur, ainsi que toute sous-combinaison de ces entiers. A titre d'exemple, les valeurs préférées pour « 1-5 » peuvent comprendre les entiers 1, 2, 3, 4, 5, 1-2, 1-3, 1-4, 1-5, 2-3, 2-4, 2-5, etc.

Tel qu'utilisé ici, le terme "environ" se réfère en particulier à une gamme de valeurs à ± 10% d'une valeur spécifique. Par exemple, le terme "environ 100" comprend les valeurs de 100 ± 10 %, c'est-à-dire les valeurs de 90 à 110.

Par « zircone », on entend en particulier du dioxyde de zirconium, également connu sous le nom d'oxyde de zirconium(IV), ou de composé de formule ZrO₂.

Par « rugosité Ra », on entend notamment la distance moyenne entre la ligne moyenne et les pics et les creux d'une surface donnée. Il s'agit donc notamment, pour une surface donnée, de l'écart moyen, ou moyenne arithmétique des distances entre pics et creux successifs. A titre d'exemple, la longueur d'évaluation de la rugosité peut être de 1,25 mm (correspondant en particulier à une longueur d'onde de coupure du filtre passe-haut de l'appareil de mesure de 0,25 mm), notamment pour une rugosité Ra telle que 0,02 < Ra ≤ 0,1; de 4,00 mm (correspondant en particulier à une longueur d'onde de coupure du filtre passe-haut de l'appareil de mesure de 0,80 mm), notamment pour une rugosité Ra telle que 0,1 < Ra ≤ 2; ou de 12,50 mm (correspondant en particulier à une longueur d'onde de coupure du filtre passe-haut de l'appareil de mesure de 2,50 mm), notamment pour une rugosité Ra telle que 2 < Ra ≤ 10.

"Ra" correspond ainsi en particulier à la différence entre cette distance moyenne et la "ligne centrale". De façon plus particulière, la rugosité Ra est mesurée suivant la norme ISO 4287, par exemple sur un profilomètre Dektak Stylus^{®} (Bruker).

### EXEMPLES

### Exemple 1 : préparation d'un dispositif médical implantable selon l'invention

Un dispositif médical implantable du commerce, par exemple un implant dentaire, est préparé selon les étapes suivantes :
- nettoyage à l'alcool (éthanol pur) ;
- bain dans un bac à ultrason avec de l'eau, par exemple de l'eau distillée ;
- séchage par soufflage d'air pur ;
- installation du dispositif dans la machine de PVD ;
- si nécessaire, nettoyage du dispositif par soufflage d'air pur ;
- mise sous vide de la machine de PVD ;
- si nécessaire, décapage plasma du dispositif.

Une fois le dispositif préparé comme indiqué ci-dessus, la couche constituée ou comprenant de la zircone est déposée sur ledit dispositif comme suit :
- nettoyage de la machine de PVD (notamment sablage du porte-substrat, des écrans suppresseurs, etc.) ;
- mise en place de la cible de zirconium, éventuellement yttrié ;
- mise en place du dispositif sur le porte substrat rotatif ;
- mise sous vide de la machine (par exemple 2 à 5.10⁻⁶ mbar) ;
- introduction du gaz plasmagène (par exemple 30 sccm d'argon) ;
- introduction du gaz réactif (par exemple 6 sccm d'oxygène) ;
- ajustement de la pression à l'intérieure de l'enceinte de dépôt à l'aide de la vanne de laminage (par exemple à 0.75Pa) ;
- mise en route de générateur DC pulsé (Puissance = 0.5A - Fréquence = 100Khz - Temps mort = 2µs par exemple) ;
- si nécessaire : amorçage du plasma à l'aide d'un générateur RF ;
- temps de dépôt à adapter en fonction notamment de l'épaisseur de la couche de dépôt désirée (pour 100 nm, 1h de dépôt peut être nécessaire, par exemple sur une machine non industrielle. Pour 800 nm, environ 8h de dépôt peuvent être nécessaire) ;
- fin du dépôt (par arrêt du générateur et du flux gazeux notamment) ;
- arrêt de la pompe turbo moléculaire et de la pompe primaire ;
- purge de la chambre d'élaboration ;
- récupération du dispositif selon l'invention, de préférence avec des gants.

Les paramètres indiqués ci-dessus sont donnés à titre d'exemple et peuvent être adaptées aisément par l'homme du métier, en fonction de la machine PVD utilisée.

En particulier, des dispositifs de l'invention, ont été obtenus selon le présent exemple, avec une épaisseur de zircone de 100 nm ou de 800 nm, à partir d'implants dentaires du commerce :

| **Nom du dispositif de l'invention** | **Référence de l'implant du commerce utilisé** | **Epaisseur de la couche de zircone** |
|---|---|---|
| A100 | Naturall+ de la société ETK^{®} | 100 nm |
| A800 | Naturall+ de la société ETK^{®} | 800 nm |
| B100 | Natea+ de la société ETK^{®} | 100 nm |
| B800 | Natea+ de la société ETK^{®} | 800 nm |

Chacun des 4 implants ci-dessus ont été complètement recouvert d'oxyde de zirconium sur leur totalité par pulvérisation cathodique magnétron. Pour ce faire, la cible correspondait à du zirconium pur sous forme métallique. Le gaz présent était de l'oxygène et le substrat était l'implant à pulvériser.

Ainsi par bombardement ionique sur la cible de zirconium, se forme une vapeur métallique de zirconium qui est oxydé par l'oxygène présent au cours de sa route vers l'implant. L'oxyde de zirconium se fixe alors sur l'implant.

### Exemple 2 : tests d'arrachements effectués sur les dispositifs de l'invention

Deux dispositifs de l'invention obtenus selon l'exemple 1, l'un avec une épaisseur de zircone de 100 nm, l'autre avec une épaisseur de zircone de 800 nm, ont été rayés en forme de damier, à l'aide d'un peigne diamant. Les rayures sont espacées d'environ 750µm.

Un morceau de ruban adhésif est collé sur le damier formé, puis arraché.

Le damier est alors observé au microscope optique.

Lorsque le damier observé au microscope est intact, le test est réussi. Lorsque la couche est décollée, arrachée à au moins un endroit du damier, le test est non réussi.

Pour les deux dispositifs de l'invention, la couche de zircone est parfaitement adhérente après le test. Aucune délamination n'est visible. Le test est donc réussi et l'adhérence de la couche grandement satisfaisante quant aux dispositifs de l'invention.

### Exemple 3 : Mesures de rugosité sur les dispositifs de l'invention

### Matériels et méthodes

Des coupes transversales des dispositifs de l'invention, par exemple de la partie apicale d'un implant dentaire selon l'invention, ont été réalisées à l'aide d'une micro-tronçonneuse. Des échantillons de chaque dispositif de 4 à 6 mm d'épaisseur ont ainsi été obtenus. Chacun d'entre eux a été analysé au profilomètre et au microscope électronique à balayage (MEB). Ceci permet si nécessaire de maintenir l'intégrité des dispositifs étudiés.

Le profilomètre (ou rugosimètre) permet de mesurer les déviations de 500 Å à 1 mm d'une surface. Il est composé d'un stylet en diamant qui se pose et se déplace sur l'échantillon avec une force constante. Ces données physiques sont converties en information électrique sur un ordinateur. On obtient la topographie, l'ondulation de surface et la rugosité de l'échantillon. Chaque mesure est effectuée sur trois zones différentes de chaque échantillon de dispositif. Ces mesures sont de 200 µm. Afin d'obtenir une valeur représentative de la rugosité de surface, la moyenne des trois valeurs obtenues a été calculée.

La mesure est effectuée sur un profilomètre Dektak Stylus^{®} (Bruker) suivant la norme ISO 4287.

Le microscope électronique à balayage (MEB) permet de prendre des mesures de composition atomique des dispositifs à partir des échantillons. Les mesures de composition se font par spectroscopie à rayons X à dispersion d'énergie ou energy dispersive spectroscopy (EDS) par émission de rayon X. L'impact du faisceau d'électrons sur l'échantillon produit des rayons X caractéristiques des éléments présents sur l'échantillon. On obtient un spectre de mesure de composition qualitative instantanée de l'échantillon avec également l'acquisition d'image en trois dimensions.

### Résultats

La rugosité des dispositifs A, B, C et D de l'invention, mentionnés dans l'exemple 1 a été mesurée comme indiqué ci-dessus.

Un implant de référence (hors invention), implant du commerce sans couche de zircone, a également été considéré.

Les résultats obtenus, correspondant à la moyenne de trois mesures, comme indiqué ci-dessus, montrent que la variation entre la rugosité Ra du dispositif initial et la rugosité Ra du dispositif obtenu à l'issue du procédé est inférieure ou égale à environ 25%, notamment inférieure ou égale à environ 20%, en particulier inférieure ou égale à environ 7%.

## Revendications

1. Dispositif médical implantable comprenant sur tout ou partie de sa surface une couche constituée ou comprenant de la zircone, ladite couche ayant une épaisseur comprise entre environ 50 et 2000 nm.

2. Dispositif selon la revendication 1 :
- dans lequel ladite couche a une épaisseur comprise entre environ 100 ou 200 et environ 2000 nm, en particulier entre environ 500 ou 600 et environ 800 nm ; ou
- comprenant une couche constituée ou comprenant de la zircone, ladite couche ayant sur une partie de la surface dudit dispositif une épaisseur comprise entre environ 50 et environ 2000 nm, notamment entre environ 50 et environ 1200 nm, et sur une autre partie de la surface dudit dispositif une épaisseur comprise entre environ 50 et environ 2000 nm, notamment entre environ 800 et environ 2000 nm.

3. Dispositif selon l'une quelconque des revendications précédentes, dans lequel la zircone est de la zircone yttriée, et/ou partiellement ou totalement dans sa phase quadratique, cubique ou monoclinique, notamment monoclinique.

4. Dispositif selon l'une quelconque des revendications précédentes, dans lequel la couche comprend, outre la zircone, au moins un élément ou composé comprenant un élément choisi parmi Mg, Ca, Ag, et Zn, en particulier sous forme métallique ou d'oxyde, se trouvant, notamment, au moins partiellement à la surface de la couche, notamment sur 1 à 100%, par exemple sur 10 à 30%, de ladite surface.

5. Dispositif selon l'une quelconque des revendications précédentes, lequel, notamment sa surface, est constitué ou comprend du titane ou de l'aluminium, le titane étant notamment du titane grade 4, ou sous la forme d'un alliage, en particulier le titane grade 5 ou un alliage titane-niobium.

6. Dispositif selon l'une quelconque des revendications précédentes, dans lequel ladite couche a une rugosité Ra comprise entre 0 et 4,0 µm, notamment de 0,6 à 2,0 µm, en particulier de 1,0 à 1,5 µm.

7. Dispositif selon l'une quelconque des revendications précédentes, lequel est un implant, en particulier un implant dentaire, ou une prothèse, en particulier une prothèse de hanche, plus particulièrement une cupule, une prothèse d'épaule, ou une prothèse de genou.

8. Procédé de préparation d'un dispositif médical implantable selon l'une quelconque des revendications 1 à 7, comprenant une étape de dépôt, sur tout ou partie de la surface d'un dispositif médical implantable, d'une couche constituée ou comprenant de la zircone, ladite couche ayant une épaisseur comprise entre environ 50 et environ 2000 nm, ledit dépôt étant effectué par dépôt physique en phase vapeur (PVD) ou par projection dynamique par gaz froid (cold spray).

9. Procédé selon la revendication 8, dans lequel le dépôt physique en phase vapeur est réalisé par pulvérisation cathodique magnétron.

10. Procédé selon l'une quelconque des revendications 8 à 9, dans lequel ledit dispositif médical implantable est en rotation, ou non, autour d'au moins un axe de rotation pendant tout ou partie du dépôt.

11. Procédé selon l'une quelconque des revendications 8 à 10, dans lequel le dépôt est effectué par dépôt physique en phase vapeur (PVD), en particulier par pulvérisation cathodique magnétron, et se fait en présence d'une cible en zirconium ou en zirconium yttrié.

12. Procédé selon la revendication 11, dans lequel le dépôt se fait en présence d'une cible en zirconium et d'une cible d'au moins un élément ou d'un composé comprenant au moins un élément choisi parmi Mg, Ca, Ag, et Zn.

13. Procédé selon la revendication 11, dans lequel le dépôt se fait en présence d'une cible en zirconium, et par mise en contact du dispositif avec au moins un élément ou d'un composé comprenant au moins un élément choisi parmi Mg, Ca, Ag, et Zn, par électrophorèse ou par jet de plasma, ledit au moins un élément ou au moins un composé comprenant au moins un élément choisi parmi Mg, Ca, Ag, et Zn étant introduit, en particulier sous forme de nanoparticules, dans le jet de plasma formé au cours du dépôt de zircone.

14. Procédé selon l'une quelconque des revendications 11 à 13, dans lequel ledit d'au moins un élément ou d'un composé comprenant au moins un élément choisi parmi Mg, Ca, Ag, et Zn est déposé:
- de façon concomitante à la zircone ; ou
- après que la zircone a été déposée.

15. Procédé selon l'une quelconque des revendications 8 à 14, dans lequel la variation entre la rugosité Ra du dispositif initial et la rugosité Ra du dispositif obtenu à l'issue du procédé est inférieure ou égale à environ 25%.
